# EUROPÄISCHE PATENTSCHRIFT

(11) **EP 3 837 230 B1**
(45) Veröffentlichungstag und Bekanntmachung des Hinweises auf die Patenterteilung: **28.09.2022**
(21) Anmeldenummer: 19758642.3
(22) Anmeldetag: 14.08.2019
(51) Int. Cl.: C07B 59/00, A61K 49/10, G01R 33/28, A61B 5/055

(54) **VERFAHREN UND VORRICHTUNG ZUR HYDRIERUNG UND HYPERPOLARISIERUNG VON TRACER-MOLEKÜLEN FÜR DIE MAGNETRESONANZBILDGEBUNG**
METHOD AND APPARATUS FOR HYDROGENATION AND HYPERPOLARIZATION OF TRACER MOLECULES FOR MAGNETIC RESONANCE IMAGING
PROCÉDÉ ET DISPOSITIF D'HYDROGÉNATION ET D'HYPERPOLARISATION DE MOLÉCULES DE TRAÇAGE POUR L'IMAGERIE PAR RÉSONANCE MAGNÉTIQUE

(30) Priorität: 14.08.2018 DE 102018119695
(43) Veröffentlichungstag der Anmeldung: 23.06.2021
(73) Patentinhaber: Albert-Ludwigs-Universität Freiburg, 79098 Freiburg (DE)
(72) Erfinder: SCHMIDT, Andreas, 79110 Freiburg (DE); HÖVENER, Jan-Bernd, 24105 Kiel (DE); HENNIG, Jürgen, 79100 Freiburg (DE); BERNER, Stephan, 79117 Freiburg (DE)
(74) Vertreter: Mertzlufft-Paufler, Cornelius
(86) Internationale Anmeldenummer: PCT/EP2019/071777
(87) Internationale Veröffentlichungsnummer: WO 2020/035518

(56) Entgegenhaltungen:
- INTERNATIONAL SOCIETY FOR MAGNETIC RESONANCE IN MEDICINE, ISMRM, 2030 ADDISON STREET, 7TH FLOOR, BERKELEY, CA 94704 USA, Nr. 163, 7. April 2017 (2017-04-07), XP040687731,
- GREEN R A ET AL: "The theory and practice of hyperpolarization in magnetic resonance using parahydrogen", PROGRESS IN NUCLEAR MAGNETIC RESONANCE SPECTROSCOPY, PERGAMON PRESS, OXFORD, GB, Bd. 67, 1. November 2012 (2012-11-01), Seiten 1-48, XP002714784, ISSN: 0079-6565, DOI: 10.1016/J.PNMRS.2012.03.001 [gefunden am 2012-03-15]
- RYAN E. MEWIS ET AL: "Probing signal amplification by reversible exchange using an NMR flow system", MAGNETIC RESONANCE IN CHEMISTRY, Bd. 52, Nr. 7, 6. Mai 2014 (2014-05-06), Seiten 358-369, XP55167861, ISSN: 0749-1581, DOI: 10.1002/mrc.4073

## Beschreibung

Die Erfindung betrifft ein Verfahren, bei dem hyperpolarisierbare Tracer-Moleküle für die Magnetresonanz-(MR)-Bildgebung hydriert werden, wobei die Tracer-Moleküle mit Wasserstoff in einem Reaktionsgemisch in Kontakt gebracht werden und eine Hydrierungsreaktion in dem Reaktionsgemisch ausgelöst wird.

Die Erfindung betrifft weiter ein Verfahren, bei dem die nach dem zuvor beschriebenen Verfahren hydrierten Tracer-Moleküle hyperpolarisiert werden.

Die Erfindung betrifft ferner eine Vorrichtung zur Hydrierung von hyperpolarisierbaren Tracer-Molekülen für die MR-Bildgebung. Die Vorrichtung weist einen Reaktor auf, wobei der Reaktor eine ein erstes Füllvolumen aufweisende erste Kammer umfasst, welche eine Zuleitung für Wasserstoffgas und/oder für eine Flüssigkeit aufweist. Beispielsweise kann es sich bei der ersten Kammer um eine Vorkammer handeln, in der eine Wasserstofflösung vorgesättigt werden kann.

Die Hydrierung eines Tracer-Moleküls kann durch eine Addition von Wasserstoff an eine ungesättigte Verbindung des Tracer-Moleküls charakterisiert sein.

Der Begriff "Tracer-Molekül" wird im Rahmen der Erfindung in einem weiten Sinn verstanden. Als Tracer-Molekül wird im Rahmen dieser Erfindung nicht nur ein hyperpolarisiertes Molekül bezeichnet, sondern auch ein solches Molekül vor Hyperpolarisierung und auch vor Hydrierung. Wenn es auf den aktuellen Zustand des Moleküls ankommt, wird in den Anmeldeunterlagen üblicherweise klarstellend das Tracer-Molekül vor Hydrierung in seinem Grundzustand als "ungesättigtes Tracer-Molekül" bezeichnet (in der Fachwelt wird ein solches Molekül häufig auch als "Precursor" bezeichnet), in seinem angeregten Zustand als "angeregtes Tracer-Molekül", nach Hydrierung als "hydriertes Tracer-Molekül" und nach Hyperpolarisierung als "hyperpolarisiertes Tracer-Molekül".

Die Hyperpolarisierung von Tracer-Molekülen für die MR-Bildgebung kann dadurch charakterisiert sein, dass vor Ausführung einer MR-Messung die für die MR-Messung geeigneten oder vorgesehenen Kernspins der Tracer-Moleküle weit über das thermische Gleichgewicht hinaus ausgerichtet werden. Das thermische Gleichgewicht kann um mehrere Größenordnungen überschritten sein, wie beispielsweise um einen Faktor von 10.000 oder mehr. Dadurch kann ein gemessenes MR-Signal der hyperpolarisierten Moleküle um einen entsprechenden Faktor verstärkt werden.

Solche Verfahren und Vorrichtungen sind aus der Praxis bekannt.

Eine bekannte Methode zur Herstellung hyperpolarisierter Tracer-Moleküle ist beispielsweise die unter der Bezeichnung *Parahydrogen Induced Polarization* (PHIP) bekannte Methode.

Zur Herstellung hyperpolarisierter Tracer-Moleküle ist es bekannt, zunächst in einem Parawasserstoffumwandler Parawasserstoff anzureichern. Hierzu wird in den Umwandler Wasserstoffgas eingeleitet. Das Wasserstoffgas wird sodann bei einer tiefen Temperatur von etwa 21 Kelvin ins thermische Gleichgewicht gebracht. Wasserstoff befindet sich bei Zimmertemperatur zu etwa 25% in einem Para-Zustand und zu etwa 75% in einem Ortho-Zustand. Dieses Gleichgewicht verschiebt sich bei tiefen Temperaturen zugunsten des Para-Zustands, so dass sich bei 20 Kelvin die meisten Wasserstoffmoleküle im Para-Zustand befinden. Da der Übergang vom Ortho-Zustand in den Para-Zustand jedoch quantenmechanisch nicht erlaubt ist, wird zur Beschleunigung der Herstellung des thermischen Gleichgewichts in dem Parawasserstoffumwandler ein Katalysator verwendet, wie beispielsweise Eisenoxid. Der so angereicherte Parawasserstoff kann sodann über eine Zeit von zwei bis drei Wochen beispielsweise in einer Flasche als Fluid, also als Gas oder unter höherem Druck und/oder bei einer tieferen Temperatur als Flüssigkeit, gelagert werden, bevor sich das thermische Gleichgewicht bei der höheren Temperatur wieder zurückbildet. Eine Lagerung kann auch unter normalen Umgebungstemperaturen erfolgen, insbesondere in der gasförmigen Phase des Parawasserstoffs.

Es ist bekannt, dass die Lagerungszeit von Parawasserstoff in Lösung reduziert ist. Beispielsweise kann die Lagerungszeit in einem organischen Lösungsmittel etwa zwei bis drei Stunden betragen. Eine Lagerung von Parawasserstoff über einen üblichen Zeitraum ist daher in einer Lösung nicht möglich. Hintergrund für die verkürzte Lagerungszeit ist, dass eine Rückkehr in den thermischen Gleichgewichtszustand in der Lösung schneller erfolgt.

Zur Herstellung hyperpolarisierter Tracer-Moleküle ist es weiter bekannt, mit dem angereicherten Parawasserstoff die Tracer-Moleküle zu hydrieren. Hierzu werden die Tracer-Moleküle mit Wasserstoff in einem Reaktor in Kontakt gebracht und eine Hydrierungsreaktion wird in dem Reaktor ausgelöst. Die Hydrierungsreaktion wird dabei üblicherweise katalytisch beschleunigt. Aus dem Stand der Technik sind verschiedene Methoden bekannt.

Nach einer ersten Methode werden zunächst in dem Reaktor die Tracer-Moleküle in gelöster Form mit einem Katalysator gemischt. Sodann wird Parawasserstoffgas in einem unteren Bereich in den Reaktor eingeleitet. Die entstehenden Blubberblasen steigen in der Lösung auf und an den Gas-Flüssigkeits-Kontaktflächen geht das Wasserstoffgas in die Lösung über. Hierdurch bildet sich ein Reaktionsgemisch. In dem gleichen Verfahrensschritt wird die Hydrierungsreaktion ausgelöst, sobald der Wasserstoff unter Anwesenheit des Katalysators ein Reaktionszentrum an einem Tracer-Molekül erreicht. Durch die Hydrierung werden Wasserstoffatome von den Tracer-Molekülen aufgenommen.

Nach einer zweiten Methode befindet sich in einem Reaktor Wasserstoffgas. Eine Flüssigkeit, in der die Tracer-Moleküle und der Katalysator gemischt sind, wird sodann in den Reaktor eingesprüht. Hierdurch bilden sich Gas-Flüssigkeits-Kontaktflächen, an denen Wasserstoffgas von der Flüssigkeit aufgenommen wird. Hierdurch wird ein Reaktionsgemisch gebildet und die Hydrierungsreaktion wird wie zuvor beschrieben noch in dem gleichen Verfahrensschritt ausgelöst, sobald Wasserstoff zu den Reaktionszentren der Tracer-Moleküle gelangt.

Zur Herstellung hyperpolarisierter Tracer-Moleküle ist es bekannt, nach oder bereits während der Hydrierung Spinordnung des von den Tracer-Molekülen aufgenommenen Wasserstoffs auf einen MR-geeigneten Atomkern des Tracer-Moleküls zu übertragen, wie beispielsweise auf einen ¹H-, ¹³C-, ¹⁵N- oder ¹⁹F-Kern. Hierzu werden die hydrierten Moleküle mit einer spin-order-transfer(SOT)-Sequenz in einem separaten Niedrigfeldpolarisator oder unmittelbar in einem für eine nachfolgende Messung verwendeten MR-Tomographen beaufschlagt. Die von der SOT-Sequenz erzeugten Magnetfeldvariationen manipulieren die Spins in Zustände, in denen sie verstärkt und gezielt Spinordnung über die J-Kopplungen austauschen, so dass am Ende der SOT-Sequenz beispielsweise eine Hyperpolarisierung eines bestimmten ¹³C-Kerns oder eines anderen MR-geeigneten Atomkerns der Moleküle erzielt wird. Beispielsweise kann eine 1-PH-INEPT+-Sequenz, eine Goldman-Sequenz oder eine ESOTHERIC-Sequenz als SOT-Sequenz angewandt werden.

Die Hyperpolarisierung eines ¹³C-Kerns, insbesondere noch bevor die Hydrierung abgeschlossen ist, kann vorteilhaft sein, da der hyperpolarisierte Zustand eines so hyperpolarisierten Tracer-Moleküls langlebiger ist und insbesondere länger andauern kann als ein geordneter Spinzustand von an die Tracer-Moleküle addiertem Parawasserstoff. Ein hyperpolarisierter Zustand von aktuell verfügbaren Tracer-Molekülen kann auf diese Weise etwa wenige Minuten aufrechterhalten bleiben, was für einige medizinische Anwendungen ausreichend sein kann.

Es kann somit auch gesagt werden, dass mit der SOT-Sequenz Spinordnung, welche im Weg der Hydrierung durch die Addition einer angereicherten Isotopenform des Wasserstoffs, wie beispielsweise Parawasserstoff, auf die Tracer-Moleküle eingebracht wird, auf ein für die MR-Bildgebung geeigneten oder für eine anschließende MR-Bildgebung gewünschten Atomkern der Tracer-Moleküle übertragen wird, so dass der Kernspin eines solchen Atomkerns ausgerichtet und somit das Tracer-Molekül hyperpolarisiert wird.

Eine medizinische Anwendung kann beispielswiese darin bestehen, die Lösung mit hyperpolarisierten Tracer-Molekülen einem Patienten zu injizieren und als Kontrastmittel zu verwenden. Ein Tracer-Molekül kann beispielsweise so ausgewählt sein, dass es bevorzugt in Tumorbereiche gelangt, so dass bei einer sich anschließenden MR-Messung das Tumorgewebe deutlicher abgebildet werden kann als anderes Gewebe. Die bevorzugte Anreicherung von Tracer-Molekülen in Tumorgewebe kann beispielsweise auch dadurch erfolgen, dass eine Perfusion infolge eines erhöhten Energiebedarfs in einer Tumorregion erhöht sein kann. Bei Verwendung von stoffwechselaktiven Tracer-Molekülen können die Tracer-Moleküle auch in einen Stoffwechselkreislauf aufgenommen werden. Ist dieser Stoffwechselkreislauf aufgrund eines Tumors krankhaft verändert, so kann dies im Wege einer MR-Bildgebung erkannt werden, sodass der Tumor erkannt und ggf. charakterisiert werden kann.

Die Methoden nach dem Stand der Technik haben unter anderem den Nachteil, dass die Anreicherung von Parawasserstoff in der Reaktionslösung für viele Anwendungen zu lange dauert. Denn nach Addition des Wasserstoffs an die Tracer-Moleküle bleiben infolge eines schnellen Zerfalls des geordneten Spinzustands nur wenige Sekunden bis zur Übertragung der Spinordnung auf einen MR-geeigneten Atomkern und anschließender Zugabe der Tracer-Moleküle zum Messobjekt sowie die Ausführung der MR-Messung.

Aus diesem Grund enthält die hydrierte Lösung von hyperpolarisierbaren und/oder hyperpolarisierten Tracer-Molekülen nur eine relativ niedrige Konzentration von Tracer-Molekülen.

Ein weiteres Problem bilden Lufteinschlüsse, welche durch das Blubbern oder Einsprühen entstehen. Hierdurch bilden sich Inhomogenitäten des von dem Polarisator erzeugten Magnetfeldes. Problematisch ist vor allem, dass infolge der Magnetfeldinhomogenitäten die T2*-Relaxationszeiten verkürzt werden. Dies führt dazu, dass bereits vor Abschluss einer Anwendung einer SOT-Sequenz ein signifikanter Teil des hyperpolarisierten Zustands der Tracer-Moleküle zerfallen ist und somit nicht mehr für eine sich anschließende Bildgebung zur Verfügung steht. Weiter bilden sich durch die Inhomogenitäten Off-Resonanzen, sodass sich bei Anwendung einer SOT-Sequenz kein optimaler Zustand ergibt, in dem die Polarisierung auf beispielsweise den ¹³C-Kern übertragen werden könnte. Ein weiteres Problem von Lufteinschlüssen ist, dass bei einer intravenösen Injektion auch Luft injiziert werden kann, was sehr problematisch für den Zustands eines Patienten sein kann und im Extremfall sogar zum Tod führen könnte.

Besonders problematisch sind die von Lufteinschlüssen entstehenden Magnetfeldinhomogenitäten bei starken Magnetfeldern wie sie beispielsweise bei der Humanbildgebung oder der Tierbildgebung verwendet werden, beispielsweise bei Magnetfeldern der Stärke 1.5 Tesla, 3 Tesla, 7 Tesla oder mehr. Der Stand der Technik ist daher insbesondere dann nachteilhaft, wenn die Hyperpolarisation nicht in einem separaten Niedrigfeldpolarisator erfolgt, sondern unmittelbar in dem MR-Tomographen. Letzteres kann vorteilhaft sein, da hierdurch ein separater Polarisator entfallen kann und auch die Zeit bis zur Applikation der Tracer-Moleküle für eine sich anschließende MR-Bildgebung reduziert wird.

Aus der Veröffentlichung Schmidt et al.: *"Long-lasting, Iiquid-state 13C hyperpolarization > 20* % *generated in an MRI system within seconds enables fast 13C imaging",* Proc. Intl. Soc. Mag. Reson. Med. 25 (2017), Nr. 0163, ist es bekannt, eine Hydrierung durch Injektion von Parawasserstoff von der Unterseite eines Reaktionsreaktors aus zu injizieren.

Aus der Veröffentlichung Green et al.: "The theory and practice of hyperpolarization in magnetic resonance using parahydrogen", PROGRESS IN NUCLEAR MAGNETIC RESONANCE SPECTROSCOPY, 67 (2012), S. 1 - 48, DOI: 10.1016/J.PNMRS.2012.03.001, ist es bekannt, die Kontaktzeit von Parawasserstoff in der Lösung durch Verlängerung der Dauer des Einblubberns oder Schüttelns des Reaktionsgemischs zu verlängern.

Aus der Veröffentlichung Mewis et al.: "Probing signal amplification by reversible exchange using an NMR flow system", MAGNETIC REONANCE IN CHEMISTRY, 52 (2014), Seiten 358-369, DOI: 10.1002/mrc.4073, ist es bekannt, Parawasserstoff in gasförmigem Zustand in ein Reaktionsgemisch einzublubbern.

Den Methoden, die in den zuvor genannten Veröffentlichungen beschrieben sind, ist gemeinsam, dass bereits durch die Herstellung der Wasserstofflösung unmittelbar die Hydrierungsaktion ausgelöst wird.

Vor dem Hintergrund des zuvor Beschriebenen ist es Aufgabe der vorliegenden Erfindung, eine schnellere Hydrierung von hyperpolarisierbaren Tracer-Molekülen für die MR-Bildgebung zu schaffen und/oder zu verhindern, dass sich Lufteinschlüsse bilden in der hydrierten Lösung. Eine weitere Aufgabe der vorliegenden Erfindung besteht darin, die Konzentration der hydrierten Moleküle in der hydrierten Lösung zu erhöhen.

Zur Lösung dieser Aufgabe schlägt die Erfindung die Merkmale von Anspruch 1 vor. Insbesondere wird somit erfindungsgemäß zur Lösung der genannten Aufgabe ein Verfahren vorgeschlagen, bei dem Tracer-Moleküle für die MR-Bildgebung hydriert werden.

Es kann sich bei den Tracer-Molekülen um beliebige hydrierbare Moleküle handeln, welche als Tracer oder Kontrastmittel für die MR-Bildgebung verwendet werden können. Dies können beispielsweise sein 2-hydroxyehtyl 1-¹³C, 2,3,3-D₃ Akrylat (HEA) bzw. nach Hydrierung 2-hydroxyehtyl 1-¹³C, 2,3,3-D₃ Proprionat (HEP) oder 1-¹³C,2,3-D₂ Fumarat bzw. nach Hydrierung 1-¹³C,2,3-D₂ Succinat oder 2,2,3,3-tetrafluoropropyl 1-¹³C, 2,3,3-D₃ Akrylat (TFPA) bzw. nach Hydrierung 2,2,3,3-tetrafluoropropyl 1-¹³C, 2,3,3-D₃ Proprionat (TFPP) oder VinylAcetat bzw. nach Hydrierung Ethyl-Acetat oder Vinyl-Pyruvat bzw. nach Hydrierung Ethyl-Pyruvat. Von Vorteil ist bei diesem Verfahren insbesondere, dass die Kontrastmittellösungen ohne einen potentiell schädlichen Katalysator herstellbar sind.

MR-Bildgebung wird im Rahmen dieser Erfindung weit verstanden und umfasst neben tomographischen MR-Verfahren auch spektroskopische MR-Verfahren jeglicher Art. Vorzugsweise handelt es sich bei der MR-Bildgebung um eine solche an einem Organismus wie an einem Menschen oder an einem Tier. Erfindungsgemäß werden die Tracer-Moleküle mit Wasserstoff in einem Reaktionsgemisch in Kontakt gebracht und eine Hydrierungsreaktion wird in dem Reaktionsgemisch ausgelöst. Das Auslösen der Hydrierungsreaktion hat zur Folge, dass die Tracer-Moleküle hydriert werden. Die Hydrierungsreaktion kann auch dadurch charakterisiert sein, dass eine Addition des Wasserstoffs an die Tracer-Moleküle erfolgt. Beispielsweise wird durch die Hydrierung das unhydrierte, ungesättigte Tracer-Molekül 2-hydroxyehtyl 1-¹³C, 2,3,3-D₃ Akrylat hydriert zu dem hydrierten Tracer-Molekül 2-hydroxyehtyl 1-¹³C, 2,3,3-D₃ Proprionat. Das Reaktionsgemisch kann dadurch charakterisiert sein, dass es ein homogenes oder heterogenes Gemisch ist, welches alle an der Hydrierungsreaktion beteiligten Stoffe umfasst. Die Stoffe können hierzu alle in einer Flüssigkeit gelöst sein, sie können emulgiert sein oder auch auf andere Weise in Kontakt zueinander stehen wie beispielsweise eine Lösung in einem Reaktor mit einem Bereich, auf dem einer der reagierenden Stoffe in fester Form aufgebracht ist. Beispielsweise kann sich in dem Reaktor ein Katalysator auch in fester Form befinden oder heterogen beigemischt sein. Der Begriff der Emulsion wird im Rahmen dieser Erfindung weit verstanden und umfasst neben einer Emulsion im eigentlichen Sinn auch eine Suspension oder einen Schaum.

Das Reaktionsgemisch besteht typischerweise aus den Tracer-Molekülen, einem Katalysator, Wasserstoff und einem Lösungsmittel wie beispielsweise Wasser. Wie weiter unten noch genauer ausgeführt, braucht das Reaktionsgemisch nicht zwingend einen Katalysator enthalten. Es kann sein, dass in dem Reaktionsgemisch die Tracer-Moleküle und der Katalysator einen Komplex mit einem Reaktionszentrum bilden. Der Komplex kann beispielsweise als Reaktionszentrum ein Rhodiumatom haben. Der Katalysator kann das Rhodiumatom enthalten. Beispielsweise kann der Katalysator hergestellt werden aus einer Mischung eines Biphosphin-Liganden wie etwa 1,4-bis-[(phenyl-3-propane sulfonate) phospine] butane disodium salt mit einem Rhodium-Komplex wie etwa bis(norbornadiene)rhodium (I) tetrafluoroborate.

Erfindungsgemäß wird in einem ersten Verfahrensschritt der Wasserstoff in einer Flüssigkeit gelöst, so dass eine Wasserstofflösung mit einem Sättigungsfaktor von mindestens 50% hergestellt wird. Bei der Flüssigkeit kann es sich um Wasser oder um ein anderes zum Lösen von Wasserstoffgas geeignetes Lösungsmittel handeln. Bevorzugt wird der Wasserstoff unter erhöhtem Druck in der Flüssigkeit gelöst.

Bevorzugt wird der zu lösende Wasserstoff als ein Wasserstoffgas bereitgestellt. Dies kann beispielsweise wie bereits zuvor beschrieben erfolgen. Das Wasserstoffgas kann in einer Isotopenform angereichert bereitgestellt werden. Als Isotope kommen neben Protium, Deuterium und Tritium in Betracht. Diese Isotope können insbesondere in ihrer Para-Form oder in ihrer Ortho-Form angereichert sein oder werden. Eine besonders bevorzugte Isotopenform ist der Parawasserstoff des Protiums (pH₂).

Weiter wird in einem, dem ersten Verfahrensschritt nachfolgenden, zweiten Verfahrensschritt die Hydrierungsreaktion ausgelöst.

Durch die Vorsättigung von Wasserstoff in einer Wasserstofflösung wird erreicht, dass Gas-Flüssigkeits-Grenzen während des Ablaufs der Hydrierungsreaktion vermieden werden. Dies hat zum einen den Vorteil, dass Lufteinschlüsse mit den weiter oben beschriebenen Nachteilen vermieden werden. Außerdem kann so der Wasserstoff sehr schnell zu den Reaktionszentren der Tracer-Moleküle gelangen, da der Wasserstoff bereits in gelöster Form vorliegt. Aufgrund einer Vorsättigung mit einem Anteil von mindestens 50% können zudem in kürzester Zeit hohe Konzentrationen von hydrierten Tracer-Molekülen in dem Reaktionsgemisch erreicht werden.

Um die Anreicherung von hydrierten Tracer-Molekülen weiter zu beschleunigen und die Konzentration von hydrierten Tracer-Molekülen weiter zu erhöhen, kann vorgesehen sein, dass der Sättigungsfaktor des Wasserstoffs in der Wasserstofflösung weiter erhöht wird. So kann vorgesehen sein, dass der der Sättigungsfaktor mindestens 80% oder mindestens 85% beträgt. Weiter bevorzugt beträgt der Sättigungsfaktor mindestens 90% oder 95%. Besonders bevorzugt beträgt der Sättigungsfaktor mindestens 98% oder 99%. Sehr vorteilhaft kann ein Sättigungsfaktor von 100% sein. Es handelt sich dann um eine vollständig gesättigte Wasserstofflösung.

Bei einer weiteren Ausgestaltung des erfindungsgemäßen Verfahrens kann vorgesehen sein, dass das Reaktionsgemisch bereits in dem ersten Verfahrensschritt hergestellt wird. Dies kann beispielsweise ohne Katalysator oder unter Verwendung eines schaltbaren Katalysators erfolgen. Der schaltbare Katalysator kann beispielsweise zunächst inaktiv sein und erst durch Einwirkung eines Aktivators wie etwa Licht oder Schall aktiviert werden. Alternativ oder zusätzlich kann vorgesehen sein, dass die Hydrierungsreaktion ausgelöst wird, indem in dem zweiten Verfahrensschritt ein schaltbarer Katalysator, beispielsweise der zuvor erwähnte schaltbare Katalysator, aktiviert wird. Solche Ausgestaltungen haben den Vorteil, dass alle erforderlichen Stoffe bereits in einem einzelnen Gefäß vermischt werden können. Die Hydrierungsreaktion kann sodann sehr rasch ablaufen, da die Reaktionsstoffe bereits miteinander vermischt sein können und eine Aktivierung des Katalysators beispielsweise mittels Belichtung, also mittels Bestrahlung mit Licht, wozu neben optischem, auch Ultraviolett-(UV)-Licht oder Infrarotlicht zählen kann, mittels Bestrahlung mit elektromagnetischer Strahlung eines anderen Spektralbereichs oder mittels Beschallung sehr schnell erfolgen kann.

Bei einer weiteren vorteilhaften Ausgestaltung des erfindungsgemäßen Verfahrens kann vorgesehen sein, dass die Hydrierungsreaktion ausgelöst wird, indem in dem zweiten Verfahrensschritt das Reaktionsgemisch bestrahlt wird. Eine derartige Ausgestaltung hat den Vorteil, dass die Hydrierungsreaktion sehr schnell und sehr gut steuerbar ablaufen kann.

Die Bestrahlung kann durch Einstrahlen elektromagnetischer Strahlung erfolgen. Die Hydrierungsreaktion kann ausgelöst werden, indem ein Katalysator durch die Bestrahlung aktiviert wird. Alternativ kann die Hydrierungsreaktion wie folgt beschrieben auch ohne Katalysator ausgelöst werden.

Bei der elektromagnetischen Strahlung kann es sich bevorzugt um optisches Licht, Ultraviolett-Licht oder Infrarot-Licht handeln. Besonders vorteilhaft kann es sein, insbesondere für die Auslösung der Hydrierungsreaktion ohne Katalysator, wenn das UV-Licht eine Wellenlänge zwischen 200nm und 250nm aufweist.

Es ist ferner bevorzugt vorgesehen, dass durch die Bestrahlung die Tracer-Moleküle in einen angeregten Elektronenzustand versetzt werden. Besonders bevorzugt erfolgt die Bestrahlung mit einer elektromagnetischen Strahlung, deren Wellenlänge derart gewählt ist, dass das Tracer-Molekül durch die Bestrahlung in den angeregten Elektronenzustand versetzt wird. Besonders bevorzugt ist die Wellenlänge so gewählt, dass ein Photon die Energie aufweist, die zur Anregung des Elektronenzustands benötigt wird. Die Anregung des Elektronenzustands des Tracer-Moleküls hat den Vorteil, dass die Reaktionsneigung des Tracer-Moleküls erhöht wird, so dass sich die Reaktionsrate mit vorhandenem Wasserstoff erhöhen kann. Derartige Ausgestaltungen haben den besonderen Vorteil, dass infolge der Entbehrlichkeit eines Katalysators die Hydrierungsreaktion sehr schnell und besonders gut steuerbar durchführbar ist. Hierdurch kann insbesondere erreicht werden, dass bei einer Übertragung von Spinordnung der Anteil hyperpolarisierter Tracer-Moleküle erhöht wird, so dass für eine sich anschließende Magnetresonanzmessung ein stärkeres Signal geliefert wird.

Besonders bevorzugt werden die Tracer-Moleküle durch die Anregung des Elektronenzustands in einen Zustand versetzt, in dem eine Aufnahmerate von Wasserstoff in die Tracer-Moleküle gegenüber dem nicht-angeregten Zustand der Tracer-Moleküle erhöht ist.

Wird als Tracer-Molekül beispielsweise ein solches mit einer Kohlenstoffdoppelbindung verwendet, wie es beispielsweise bei einem ungesättigten Fumarat der Fall ist, so wird eine Bestrahlung des Tracer-Moleküls mit UV-Licht mit einer geeigneten Wellenlänge von häufig zwischen 200nm und 250nm dazu führen, dass die Tracer-Moleküle in einen angeregten Elektronenzustand versetzt werden, bei der eine Kohlenstoffdoppelbindung angeregt und daher anschaulich betrachtet quasi aufgebrochen wird. Sollte für das betreffende Tracer-Molekül die für die Anregung des Elektronenzustands benötigte Energie außerhalb des Korridors von 200nm bis 250nm liegen, so sollte die Einstrahlung bevorzugt mit einer entsprechenden Lichtenergie außerhalb dieses Wellenbereichs erfolgen. Durch die Anregung der Kohlenstoffdoppelbindung werden die Tracer-Moleküle empfänglich für die Aufnahme von Wasserstoff. Ist in dem Reaktionsgemisch Wasserstoff vorhanden, so wird daher durch die Bestrahlung des Reaktionsgemischs die Hydrierungsreaktion ausgelöst.

Besonders bevorzugt besteht das Reaktionsgemisch ausschließlich aus einer Mischung von destilliertem Wasser, Wasserstoff und den Tracer-Molekülen. Hierdurch kann die Effizienz der Hydrierung verbessert werden, da die angeregten Tracer-Moleküle als Reaktionspartner lediglich Wasserstoff vorfinden. Ein ebenfalls bevorzugtes Reaktionsgemisch kann auch weitere Stoffe umfassen, wobei die weiteren Stoffe so beschaffen sind, dass sie nicht mit den angeregten Tracer-Molekülen reagieren. Dass die Zugabe weiterer Stoffe vorteilhaft sein kann, beispielsweise um die hydrierte Stoffmenge zu erhöhen, wurde bereits zuvor beschrieben.

Zur Beschleunigung der Hydrierung und Vermeidung von Lufteinschlüssen kann alternativ vorgesehen sein, dass das Reaktionsgemisch erst in dem zweiten Verfahrensschritt hergestellt wird. Vorzugsweise wird die Hydrierungsreaktion unmittelbar durch die Herstellung des Reaktionsgemischs ausgelöst.

Besonders einfach und schnell kann ein Verfahren sein, bei dem vorgesehen ist, dass die Hydrierungsreaktion ausgelöst wird, indem in dem zweiten Verfahrensschritt die Wasserstofflösung mit einem weiteren Reaktionsstoff oder Gemisch von Reaktionsstoffen vermischt wird. Die beiden zu vermischenden Bestandteile des hergestellten Gemischs können dabei insbesondere in jeweils einem separaten Gefäß vorliegen und durch Zuleiten eines Inhalts des einen Gefäßes in das andere Gefäß miteinander vermischt werden. Durch das Vermischen kann wie zuvor beschrieben insbesondere das Reaktionsgemisch hergestellt werden. Es kann vorgesehen sein, das die Wasserstofflösung hierbei frei von anderen an der Hydrierungsreaktion beteiligten Reaktionsstoffen ist, während das weitere Gemisch die übrigen Reaktionsstoffe umfasst. Es kann auch vorgesehen sein, dass die Wasserstofflösung ein Gemisch mit zumindest einem weiteren an der Hydrierungsreaktion beteiligten Stoff bildet und das weitere Gemisch lediglich einen oder die weiteren verbleibenden Reaktionsstoffe umfasst. So können beispielsweise im ersten Verfahrensschritt in der Wasserstofflösung die Tracer-Moleküle gelöst oder emulgiert sein oder werden und im zweiten Verfahrensschritt eine Vermischung mit dem Katalysator erfolgen. Möglich ist gleichfalls eine Vermischung der Wasserstofflösung im ersten Verfahrensschritt mit dem Katalysator und im zweiten Verfahrensschritt mit den Tracer-Molekülen. Dabei können die einzelnen Stoffe gelöst, emulgiert oder in fester Form vorliegen.

Zur weiteren Erhöhung der Hydrierungsgeschwindigkeit und/oder der hydrierten Stoffmenge kann vorgesehen sein, dass die Flüssigkeit und/oder die Wasserstofflösung und/oder das Reaktionsgemisch mit einem Stoff angereichert wird, der die Aufnahmefähigkeit der Flüssigkeit und/oder der Wasserstofflösung und/oder des Reaktionsgemischs für Wasserstoff erhöht. Eine Zugabe des Stoffes erfolgt vorzugsweise, bevor die Hydrierungsreaktion ausgelöst wird.

Die Zugabe erfolgt dabei besonders bevorzugt vor oder während eines Zeitraums, währenddessen der Wasserstoff in der Wasserstofflösung gelöst wird. So kann der Wasserstofflösung beispielsweise Ethanol und/oder Dimethylsulfoxid beigemischt sein und/oder die Wasserstofflösung kann Fluorinert oder ein Fluorinert-Gemisch sein. So kann beispielsweise durch Beimischung von 10% Ethanol zu Wasser die lösbare Wasserstoffmenge bei 50 bar auf etwa 80 mM in etwa verdoppelt werden.

Die Aufnahme von Wasserstoff in die Tracer-Moleküle kann weiter verbessert werden, wenn vorgesehen ist, dass dem Reaktionsgemisch ein H2-Donor zugegeben wird. Als H2-Donor kommen beispielsweise Cryptohphan-Käfige in Betracht. Dabei kann eine Zugabe des H2-Donors unmittelbar in das Reaktionsgemisch erfolgen oder auch mittelbar, beispielsweise indem eine Zugabe zunächst in die Wasserstofflösung erfolgt, mit der anschließend das Reaktionsgemisch hergestellt wird. Bevorzugt wird der H2-Donor nach der Hydrierungsreaktion wieder entfernt. Der H2-Donor ist bevorzugt biokompatibel ausgebildet.

Eine weitere Beschleunigung der Hydrierung kann erfolgen, wenn vorgesehen ist, dass eine weitere mit einem Katalysator, beispielsweise dem bereits zuvor genannten Katalysator, in Kontakt stehende Flüssigkeit mit einem Wasserstoffgas vorgesättigt wird. Dies kann beispielsweise eine Voraktivierung des Katalysators bewirken. Bevorzugt handelt es sich bei dem den Katalysator vorsättigenden Wasserstoffgas um normales Wasserstoffgas oder um Parawasserstoffgas. Der Katalysator kann in der weiteren Flüssigkeit gelöst oder emulgiert sein oder auch in fester Form vorliegen. Derartige Ausgestaltungen der Erfindung können insbesondere bei Katalysatoren vorteilhaft sein, welche mittels heterogener Katalyse die Hydrierungsreaktion beschleunigen oder auslösen.

Zur weiteren Beschleunigung der Hydrierungsreaktion und/oder zur Erhöhung der Konzentration hyperpolarisierter Tracer-Moleküle kann vorgesehen sein, dass in dem ersten Verfahrensschritt das Wasserstoffgas mit einem gegenüber der Atmosphäre erhöhten Druck beaufschlagt wird. Beispielsweise kann der Druck 50 bar betragen. Bevorzugt erfolgt auch die Hydrierungsreaktion unter erhöhtem Druck. Eine Erhöhung des Drucks kann bewirken, dass eine Wasserstoffsättigung der Wasserstofflösung und/oder des Reaktionsgemischs mit Wasserstoff und/oder mit Tracer-Molekülen erhöht ist. Bei 50 bar kann die in Wasser gelöste Wasserstoffkonzentration beispielsweise 40 mM betragen. Auch kann die Hydrierungsreaktion unter höheren Temperaturen erfolgen, da eine Siedetemperatur erhöht sein kann. Besonders bevorzugt ist der Druck, unter dem die Hydrierungsreaktion erfolgt, geringer als der Druck, der beim Lösen des Wasserstoffgases in der Wasserstofflösung vorliegt. Ein solcher Druckgradient kann vorteilhaft sein, um das Reaktionsgemisch möglichst effektiv herstellen zu können.

Es kann vorgesehen sein, dass die unter erhöhtem Druck ablaufende Hydrierungsreaktion unterhalb einer tatsächlichen Siedetemperatur und oberhalb einer Atmosphärendruck-Siedetemperatur erfolgt. Die Siedetemperatur ist dabei insbesondere auf eine Flüssigkeit bezogen, in der die Hydrierungsreaktion abläuft, beispielsweise die bereits zuvor genannte Flüssigkeit oder weitere Flüssigkeit. Bevorzugt erfolgt die Hydrierungsreaktion oberhalb von 100°C. Es ist darauf zu achten, dass ein Katalysator gewählt wird, der dieser Temperatur standhält. Die Erhöhung der Temperatur hat den Vorteil, dass die Hydrierungsreaktion beschleunigt erfolgt. Wegen des erhöhten Drucks kann die tatsächliche Siedetemperatur über die Atmosphärendruck-Siedetemperatur, das ist die Siedetemperatur bei Atmosphärendruck, steigen. Die tatsächliche Siedetemperatur sollte im Regelfall nicht überschritten werden, da andernfalls große Blubberblasen entstehen können, welche zu Lufteinschlüssen führen können.

Es kann weiter vorgesehen sein, dass bei oder nach Auslösen der Hydrierungsreaktion der Druck in einer Kammer eines Reaktors, in der die Hydrierungsreaktion stattfindet, beispielsweise in dem bereits zuvor genannten Gefäß, verringert wird, sodass Wasserstoff infolge Übersättigung unter Blasenbildung austritt. Alternativ oder zusätzlich kann die Verringerung des Drucks so erfolgen, dass eine Siedetemperatur eines Stoffs, welcher dem Reaktionsgemisch zugegeben ist, und/oder eine Siedetemperatur des Reaktionsgemischs überschritten wird. Eine Blasenbildung könnte vorteilhaft sein, da die in dem Reaktionsgemisch aufsteigenden Blasen ein Wasserstoffreservoir bilden können und die Hydrierungsreaktion intensivieren können. Ein Verdampfen kann ferner vorteilhaft sein, um die Konzentration hydrierter und/oder hyperpolarisierter Tracer-Moleküle zu erhöhen.

Es kann ferner vorgesehen sein, dass nach Auslösen der Hydrierungsreaktion das Reaktionsgemisch mit einem Wasserstoffgas in Kontakt gebracht wird. Bevorzugt ist dieses Wasserstoffgas in einer Isotopenform wie zuvor beschrieben angereichert wie etwa als Parawasserstoffgas. Die zuvor beschriebene Inkontaktbringung kann beispielsweise in Kombination mit und/oder unter Anwendung einer an sich bekannten Methode wie der bereits oben beschriebenen Blubbermethode oder Einsprühmethode erfolgen. Die Kombination dieser Methoden kann dazu führen, dass die Hydrierungsreaktion zum einen noch schneller verläuft, wobei zugleich auch hohe Konzentrationen von hydrierten und/oder hyperpolarisierten Tracer-Molekülen herstellbar sind.

Es kann weiter vorgesehen sein, dass die Hydrierungsreaktion in einem Volumen innerhalb eines Polarisators erfolgt. Bei dem Polarisator kann es sich beispielsweise um die weiter unten beschriebenen Varianten eines Polarisators handeln. Es kann ferner vorgesehen sein, dass während eines Zeitraums, währenddessen die Hydrierungsreaktion abläuft, Spinordnung auf einen MR-geeigneten Atomkern von bereits hydrierten Tracer-Molekülen übertragen wird. Die Übertragung der Spinordnung kann beispielsweise erfolgen wie an anderer Stelle in dieser Beschreibung dargelegt.

Zur Lösung der genannten Aufgabe sind erfindungsgemäß die Merkmale eines nebengeordneten Anspruchs vorgesehen, welcher auf ein Verfahren gerichtet ist, bei dem Tracer-Moleküle für die Magnetresonanzbildgebung hyperpolarisiert werden. Insbesondere wird zur Lösung der genannten Aufgabe somit erfindungsgemäß bei einem solchen Verfahren vorgeschlagen, dass bei der Ausführung des Verfahrens ein Verfahren angewendet wird, bei dem hyperpolarisierbare Tracer-Moleküle für die Magnetresonanzbildgebung hydriert werden in einer Weise wie zuvor beschrieben.

Die Übertragung von Spinordnung auf einen MR-geeigneten Atomkern der Tracer-Moleküle kann nach Methoden erfolgen, wie sie dem Fachmann bekannt sind, beispielsweise mittels der bereits oben beschrieben spin-order-transfer-Sequenz in einem Polarisator. Bei dem Polarisator kann es sich um einen Niedrigfeldpolarisator handeln, der nicht mit dem MR-Gerät identisch ist, mit dem die spätere MR-Messung eines Messobjekts durchgeführt wird. Bei dem Polarisator kann es sich auch um das MR-Gerät handeln, mit dem die MR-Messung eines Messobjekts, insbesondere eines Versuchstiers oder eines Patienten, durchgeführt werden soll. Das MR-Gerät kann ein Hochfeldpolarisator sein mit einer Feldstärke des Hauptmagnetfelds von mindestens 1 Tesla, mindestens 3 Tesla oder mindestens 7 Tesla.

Die Übertragung von Spinordnung innerhalb der Tracer-Moleküle erfolgt bevorzugt in einem Zeitraum, der beginnt, sobald oder nachdem die Hydrierungsreaktion ausgelöst wird. Dieser Zeitraum kann daher auch noch nach Ablauf der Hydrierungsreaktion beginnen.

Zur Lösung der genannten Aufgabe sind erfindungsgemäß die Merkmale des auf eine Vorrichtung gerichteten nebengeordneten Anspruchs vorgesehen. Insbesondere wird zur Lösung der genannten Aufgabe somit erfindungsgemäß bei einer Vorrichtung der eingangs beschriebenen Art vorgeschlagen, dass Mittel ausgebildet sind, mit denen ein Verfahren ausführbar ist, bei dem hyperpolarisierbare Tracer-Moleküle für die Magnetresonanzbildgebung hydriert werden, wobei das Verfahren erfindungsgemäß, insbesondere wie zuvor beschrieben und/oder nach einem der auf ein entsprechendes Verfahren gerichteten Schutzansprüche, ausgebildet ist. Bevorzugt ist die Vorrichtung eingerichtet, ein solches erfindungsgemäßes Verfahren auszuführen.

Bei einer Ausgestaltung der erfindungsgemäßen Vorrichtung kann vorgesehen sein, dass eine ein weiteres Füllvolumen aufweisende zweite Kammer ausgebildet ist, wobei die erste Kammer und die zweite Kammer über eine Verbindungsleitung miteinander in Verbindung stehen. Bei alternativen Ausgestaltungen können auch weitere Kammern vorgesehen sein.

Es können ein Heizelement, vorzugsweise zur Einstellung einer Temperatur, bei der die Hydrierungsreaktion abläuft, und/oder Mittel zum Einstellen eines Drucks in der ersten und/oder in der zweiten Kammer ausgebildet sein. Die Mittel zum Eistellen eines Drucks können beispielsweise ein oder mehrere Ventile umfassen. Die Einstellbarkeit einer Temperatur und/oder bestimmter Drücke kann, wie bereits zuvor beschrieben, vorteilhaft sein.

Die Erfindung wird nun anhand einiger weniger Ausführungsbeispiele näher beschrieben, ist jedoch nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen wie sie zuvor beschrieben worden sind.

Es zeigt:
- Fig. 1: ein Flussdiagramm eines Ausführungsbeispiels eines erfindungsgemäßen Verfahrens, bei dem hyperpolarisierbare Tracer-Moleküle für die MR-Bildgebung hydriert und hyperpolarisiert werden.
- Fig. 2: ein Ausführungsbeispiel einer Vorbereitung eines Katalysators, welcher für die Ausführung des in Fig. 1 gezeigten Verfahrens verwendbar ist,
- Fig. 3: ein Ausführungsbeispiel einer erfindungsgemäßen Hydrierung und Hyperpolarisierung eines Tracer-Moleküls für die MR-Bildgebung, wobei der in Fig. 2 gezeigte Katalysator verwendet wird,
- Fig. 4: ein Ausführungsbeispiel einer erfindungsgemäßen Vorrichtung zur Hydrierung von hyperpolarisierbaren Tracer-Molekülen für die Magnetresonanzbildgebung,
- Fig. 5: ein alternatives Ausführungsbeispiel einer erfindungsgemäßen Vorrichtung zur Hydrierung von hyperpolarisierbaren Tracer-Molekülen für die Magnetresonanzbildgebung,
- Fig. 6: ein für eine MR-Messung an einem Patienten vorbereitete Messvorrichtung, in der eine Vorrichtung, wie sie in Fig. 4 oder Fig. 5 gezeigt ist, angeordnet ist.
- Fig. 7: ein alternatives Ausführungsbeispiel einer erfindungsgemäßen Hydrierung und Hyperpolarisierung eines Tracer-Moleküls für die MR-Bildgebung durch photochemische Reaktion und ohne Verwendung eines Katalysators.

Bei der nachfolgenden Beschreibung verschiedener Ausführungsbeispiele der Erfindung erhalten in ihrer Funktion übereinstimmende Elemente auch bei abweichender Gestaltung oder Formgebung übereinstimmende Bezugszahlen.

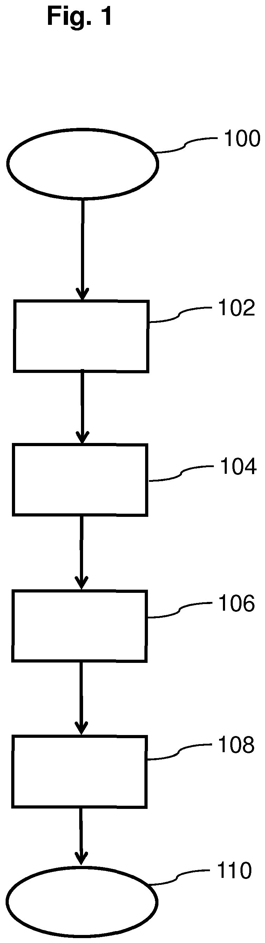

Fig. 1 zeigt ein Flussdiagramm eines Ausführungsbeispiel eines Verfahrens, bei dem hyperpolarisierbare Tracer-Moleküle für die MR-Bildgebung in erfindungsgemäßer Weise hydriert und hyperpolarisiert werden.

Das Verfahren wird zunächst in Schritt 100 initialisiert.

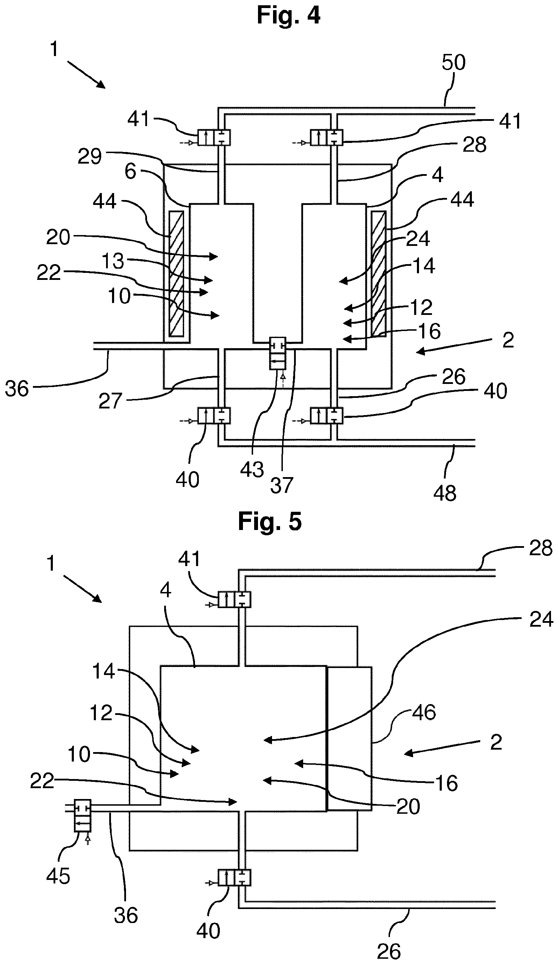

Hierbei wird die zur Durchführung des Verfahrens benötigte Apparatur bereitgestellt und hergerichtet. Eine mögliche Ausgestaltung einer solchen Apparatur ist in Fig. 4 bis Fig. 6 dargestellt und wird weiter unten genauer beschrieben. Bei der Herrichtung der Apparatur wird in dem hier beschriebenen Ausführungsbeispiel eine mit Parawasserstoff gefüllte Flasche 30 an einen Reaktor 2 angeschlossen. Weiter werden an den Reaktor 2 Quellen angeschlossen, welche die weiteren für die Durchführung des Verfahrens benötigten Stoffe bereitstellen wie insbesondere den Katalysator 22, 86 und die noch ungesättigten Tracer-Moleküle 20, 88. Soll sich nach Beendigung des Verfahrens eine MR-Messung eines Patienten 34 anschließen, so kann ein Patient 34 in einen MR-Tomographen 32 gelegt werden, es kann eine Kanüle 38 gelegt werden und die Kanüle 38 kann mit einer ausgehenden Leitung 36 des Reaktors 2 verbunden werden. Bei der Initialisierung des Verfahrens werden in dem hier beschriebenen Ausführungsbeispiel weiter Anfangsstellungen der Ventile 40, 41, 43, 45 eingestellt.

Nach der Initialisierung des Verfahrens wird in Schritt 102 Wasserstoffgas 18 in Form von Parawasserstoffgas bereitgestellt. Dies kann beispielsweise erfolgen, indem ein Ventil 42 der mit Parawasserstoffgas befüllten Falsche 30 geöffnet wird. Alternativ kann beispielsweise auch vorgesehen sein, dass das Parawasserstoffgas bereitgestellt wird, indem in einem Parawasserstoffumwandler zunächst der Parawasserstoff hergestellt wird, beispielsweise wie bereits oben beschrieben.

In einem nächsten Schritt 104 werden sodann später für die Hydrierungsreaktion benötigten Stoffgemische bereitgestellt und/oder hergestellt. Wie im Ausführungsbeispiel beschrieben, wird zunächst in einer ersten Kammer 4 des Reaktors 2 eine Wasserstofflösung 14 hergestellt, indem das in Schritt 102 bereitgestellte Parawasserstoffgas in die erste Kammer 4 geleitet wird, wobei sich in der ersten Kammer 4 Wasser befindet. Beispielsweise kann hierzu 5 Minuten lang das Parawasserstoffgas in das Wasser geblubbert werden. In einem alternativen Ausführungsbeispiel befindet sich in der Kammer 4 ein anderes Lösungsmittel, welches mit 10% Ethanol versehen ist. Ferner wird in Schritt 104 in einer zweiten Kammer 6 des Reaktors 2 ein weiteres Stoffgemisch hergestellt, indem die hyperpolarisierbaren Tracer-Moleküle 20, 88 für die MR-Bildgebung sowie der Katalysator 22 bereits in Wasser gelöst oder emulgiert in Kammer 6 geleitet wird oder indem diese Stoffe einer in Kammer 6 befindlichen weiteren Flüssigkeit 13, bei der es sich um Wasser handeln kann, zugegeben werden. In einem alternativen Ausführungsbeispiel werden die Tracer-Moleküle 20, 88 mit der Wasserstofflösung 14 in der Kammer 4 gemischt, bevor dieses Gemisch in die Kammer 6 zur Hydrierung geleitet wird.

In Schritt 106 werden sodann die in Schritt 104 bereitgestellten und/oder hergestellten Stoffgemische miteinander vermischt. Hierzu wird ein zwischen den Kammern 4 und 6 befindliches Ventil 43 einer Verbindungsleitung 37 geöffnet. Hierdurch strömt die Wasserstofflösung 14 über die die erste Kammer 4 mit der zweiten Kammer 6 verbindende Verbindungsleitung 37 aus der ersten Kammer 4 in die zweite Kammer 6, da die erste Kammer 4 mit einem höheren Druck beaufschlagt wird als die zweite Kammer 6. Durch das Vermischen der Stoffgemische wird die Hydrierungsreaktion ausgelöst und die Tracer-Moleküle 20 werden hydriert.

Nach Auslösen der Hydrierungsreaktion wird in Schritt 108 Spinordnung von den an die Tracer-Moleküle 20 addierten Wasserstoffatomen auf einen MR-geeigneten Atomkern der Tracer-Moleküle 20 übertragen. Hierzu wird der als MR-Tomograph 32 ausgebildete Polarisator mit einer SOT-Sequenz angesteuert. Die hierdurch hergestellten hyperpolyarisierten Tracer-Moleküle 20 stehen sodann zur weiteren Verwendung an der ausgehenden Leitung 36 des Reaktors 2 bereit.

In Schritt 110 wird das Verfahren sodann beendet. Hieran kann sich eine MR-Messung, welche mittels des MR-Tomographen 32 durchgeführt wird, anschließen. Hierzu wird ein die Leitung 36 verschließendes Ventil 45 geöffnet, so dass die hyperpolarisierten Tracer-Moleküle 20 über die Kanüle 38 in den Blutkreislauf des Patienten 34 gelangen können, so dass die Tracer-Moleküle 20 als Kontrastmittel für die sich anschließende MR-Messung verwendet werden können.

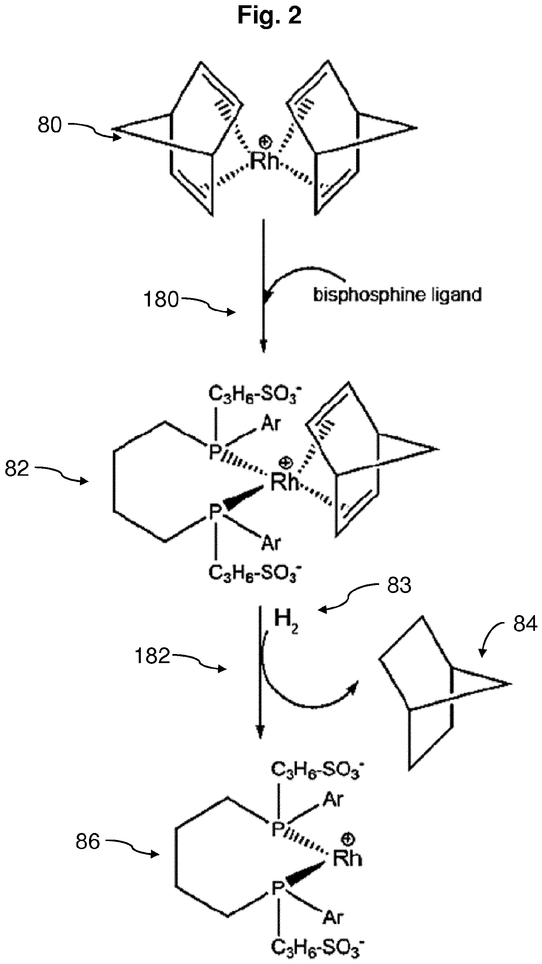

Fig. 2 zeigt ein Ausführungsbeispiel einer Vorbereitung eines Katalysators 22, 86, welcher für die Ausführung des in Fig. 1 gezeigten Verfahrens verwendbar ist.

Ausgangspunkt ist eine Komplex 80, der aus zwei Norbornadien und einem zentralen Rhodiumatom besteht. Dieser Komplex 80 wird zunächst gelöst, beispielsweise in Aceton oder in warmem Wasser. Sodann wird in Schritt 180 ein Bisphosphin-Ligand zu der Lösung hinzugegeben. Hierdurch wird eine Austauschreaktion 180 ausgelöst, bei der eines der beiden Norbornadien ausgetauscht wird durch das Bisphosphin. Hierdurch entsteht ein Komplex 82, der aus einem Norbornadien-Molekül, einem Bisphosphin-Liganden und einem zentralen Rhodiumatom besteht. Anschließend wird in Schritt 182 das verbleibende Norbornadien-Molekül 84 durch Zugabe von normalem Wasserstoffgas 83 von dem Komplex 82 abgespalten, so dass der Katalysator 86 zur weiteren Verwendung hergestellt ist. Beispielsweise kann so eine Katalysatorlösung mit einer Katalysatorkonzentration von 20 mM hergestellt werden.

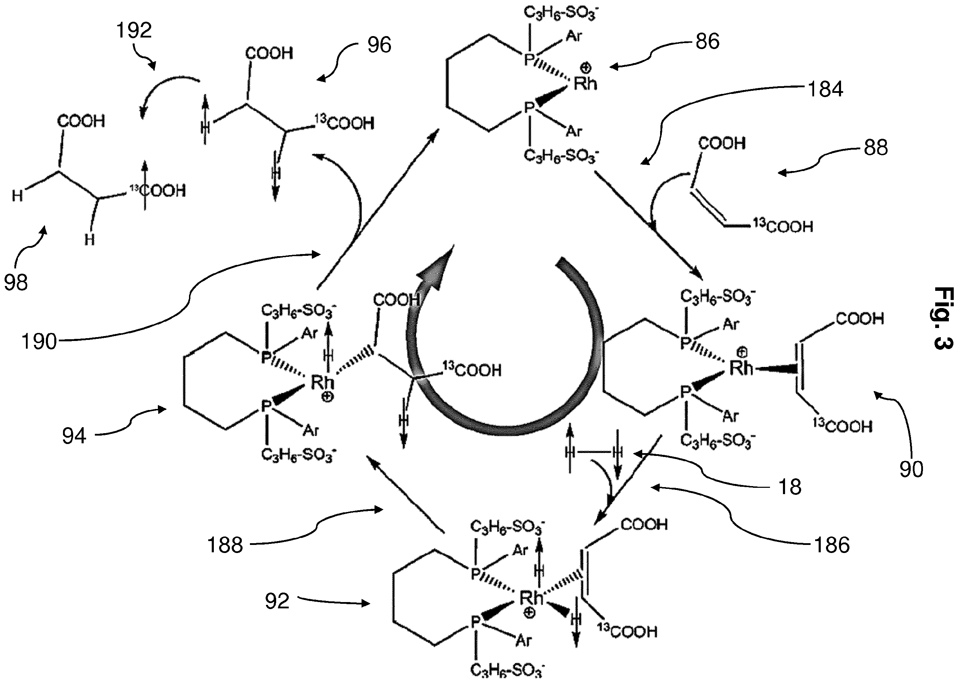

Fig. 3 zeigt eine erfindungsgemäße Hydrierung und Hyperpolarisierung eines hyperpolarisierbaren Tracer-Moleküls 20, 88 bis 98 für die MR-Bildgebung, wobei der in Fig. 2 gezeigte Katalysator 22, 86 verwendet wird.

Hierzu werden zunächst in Schritt 184 der vorbereiteten Katalysatorlösung, welche den Katalysator 86 enthält, ungesättigte Tracer-Moleküle 88 zugegeben, welche in dem hier beschriebenen Ausführungsbeispiel ein Fumarat mit einem ¹³C-Kern bildet. Hierdurch wird ein Komplex 90 erzeugt, der aus dem Katalysator 86, dem ungesättigten Tracer-Molekül 88 und einem zentralen Rhodiumatom besteht. Die Lösung kann beispielsweise eine Konzentration von Tracer-Molekülen 20, 88 in Höhe von 50 mM haben.

In einem sich nachfolgenden Schritt, der dem zuvor beschriebenen Schritt 106 entspricht, wird der Lösung mit dem Komplex 90 in Schritt 186 eine Wasserstofflösung 14 zugegeben, in der das als Parawasserstoffgas ausgebildete Wasserstoffgas 18 gelöst ist. Hierdurch bildet sich zunächst ein Komplex 92, der aus dem ungesättigten Tracer-Molekül 88, dem Katalysator 86 und einem zentralen Rhodiumatom mit addiertem Parawasserstoff besteht. Sodann erfolgt die eigentliche Hydrierungsreaktion 188, bei der die Parawasserstoffatome von dem und/oder über das Rhodiumatom zu dem Tracer-Molekül 20, 88, 96 wandern. Hierdurch entsteht ein Komplex 94 aus dem hydrierten Tracer-Molekül 96, dem Katalysator 86 und einem zentralen Rhodiumatom. Sodann spaltet sich in Schritt 190 das hydrierte Tracer-Molekül 96 von dem Katalysator 86 ab. Damit ist die Hydrierung des Tracer-Moleküls 20, 88 bis 98 abgeschlossen.

In einem sich anschließenden Schritt 192 der dem bereits zuvor beschriebenen Schritt 108 entspricht, wird sodann das Tracer-Molekül 20, 96, 98 mittels Transfers von Spinordnung auf den ¹³C-Kern hyperpolarisiert, sodass am Ende dieses Prozesses ein hyperpolarisiertes Tracer-Molekül 98 geschaffen ist.

Das in Fig. 2 und Fig. 3 Gezeigte stellt lediglich ein konkretes Beispiel dar. Es können auch andere Arten von Katalysatoren 22, Tracer-Molekülen 20 und Variationen im Verfahrensablauf verwendet oder angewandt werden.

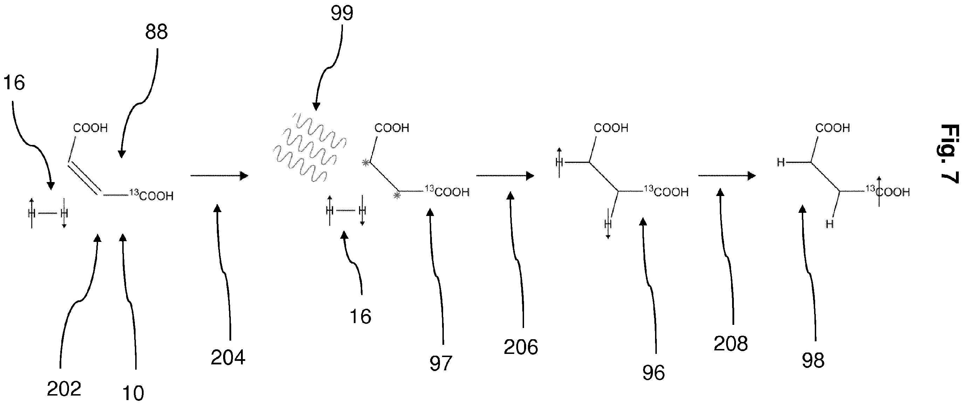

Eine vorteilhafte Variante des Verfahrens, bei der ein Katalysator 86 entbehrlich ist, ist in Fig. 7 dargestellt. In einem ersten Schritt 202 wird zunächst ein Reaktionsgemisch 10 hergestellt, das Wasserstoff 16 und ungesättigte Tracer-Moleküle 88 umfasst.

Zur Herstellung des Reaktionsgemischs kann insbesondere die weiter unten genauer beschriebene Hydrierungsvorrichtung 1 gemäß Fig. 5 verwendet werden, aber auch die Vorrichtung 1 gemäß Fig. 4. Die Vorrichtung 1 gemäß Fig. 5 weist lediglich eine einzelne Kammer 4 auf, in die eine Lösung eingebracht wird, in der die ungesättigten Tracer-Moleküle 88 und der Wasserstoff 16 gelöst und/oder emulgiert sind.

Der Wasserstoff 16 kann hierbei beispielsweise mittels Einblubberns eines Wasserstoffgases 18 zu der Lösung zugefügt werden oder auch bereits vorher in einer separaten Flüssigkeit gelöst sein, die dann in die Kammer 4 zugefügt wird. Hierzu könnte auch eine Vorrichtung 1 mit zwei Kammern 4 wie sie beispielsweise in Fig. 4 gezeigt ist, verwendet werden.

Die Reihenfolge des Einbringens der vorzugsweise gelösten oder emulgierten ungesättigten Tracer-Moleküle 88 und des Einbringens des Wasserstoffs 16 kann je nach Ausgestaltung des Verfahrens unterschiedlich sein.

Vorbereitende Schritte können wie bereits zuvor beschrieben durchgeführt werden wie beispielsweise die zuvor beschriebene Bereitstellung des Wasserstoffgases als Parawasserstoffgas.

Nach Herstellung des Reaktionsgemischs in Schritt 202 mit einer mindestens 50%-igen Wasserstoffvorsättigung wird sodann ohne weitere Zugabe von Wasserstoffgas 18 oder auch mit weiterer Zugabe von Wasserstoffgas 18 in einem nachfolgenden Schritt 204 das Reaktionsgemisch 18 mit elektromagnetischer Strahlung bestrahlt, so dass die ungesättigten Tracer-Moleküle 88 in einen angeregten Elektronenzustand überführt werden und somit angeregte Tracer-Moleküle 97 bilden. Hierzu kann je nach Ausgestaltung der Tracer-Moleküle 88 beispielsweise UV-Licht mit einer Wellenlänge zwischen 200nm und 250 nm verwendet werden. Beispielsweise kann durch die Bestrahlung von Tracer-Molekülen 88 mit einer Kohlenstoffdoppelbindung, wie etwa bei einem Fumarat, die Kohlenstoffdoppelbindung in einen angeregten Zustand gebracht werden.

So angeregte Tracer-Moleküle 97 werden durch diese Anregung hochreaktiv und suchen sich einen Reaktionspartner. Da in dem Reaktionsgemisch 10 Wasserstoff 16 vorhanden ist, wird der Wasserstoff 16 von den angeregten Tracer-Molekülen 97 aufgenommen, so dass eine Hydrierungsreaktion 206 ausgelöst wird. In Fig. 7 sind die angeregten Elektronen durch ein Sternchen-Symbol dargestellt. In dem speziellen, in Fig. 7 gezeigten Ausführungsbeispiel werden Elektronen der an der Kohlenstoffdoppelbindung beteiligten Kohlenstoffatome angeregt. Von der Erfindung sind auch andere Anregungszustände der jeweils verwendeten Tracer-Moleküle 88, 97 umfasst.

Infolge der Hydrierung werden hydrierte Tracer-Moleküle 96 gebildet.

Die hydrierten Tracer-Moleküle 96 können sodann wie bereits zuvor beschrieben in Schritt 208 mittels Transfers von Spinordnung an einem 13C-Kern des hydrierten Tracer-Moleküls 96 hyperpolarisiert werden.

Fig. 4 zeigt ein Ausführungsbeispiel einer erfindungsgemäßen Vorrichtung 1 zur Hydrierung von hyperpolarisierbaren Tracer-Molekülen 20, 88 bis 98 für die MR-Bildgebung.

Die Vorrichtung 1 umfasst einen Reaktor 2 mit einer ersten Kammer 4 und einer zweiten Kammer 6. Der Reaktor 2 hat einen Einlass 48, der sich verzweigt in eine Zuleitung 26 der ersten Kammer 4 und eine Zuleitung 27 der zweiten Kammer 6. Ausgangsseitig hat der Reaktor 2 an einer oberen Seite einen Auslass 50, der einen Auslass 28 der ersten Kammer 4 und einen Auslass 29 der zweiten Kammer 6 vereint. Im unteren Bereich der zweiten Kammer 6 hat diese ausgangsseitig ferner eine ausgehende Leitung 36. Diese Leitung 36 ist beispielsweise mit einem Injektionskatheter verbindbar. Weiter weist der Reaktor 2 Heizelemente 44 auf, mit denen eine erste Temperatur in der ersten Kammer 4 und einer zweiten Temperatur in der zweiten Kammer 6 einstellbar ist. Beispielsweise kann die erste Temperatur 75 °C betragen und die zweite Temperatur 60 °C. Weiter weist der Reaktor 2 mit den Ventilen 41 der Auslässe 28, 29 Mittel auf, um in der ersten Kammer 4 einen ersten Druck und in der zweiten Kammer 6 einen zweiten Druck einzustellen, wobei die Drücke jeweils durch den eingangsseitig am Einlass 48 eingestellten Druck begrenzt sind.

An den Anschlüssen der ersten Kammer 4 und zweiten Kammer 6 sind steuerbare Ventile 40, 41, 43, 45 angeordnet. Mit den Ventilen 40 der Zuleitung 26 der ersten Kammer 4 und der Zuleitung 27 der zweiten Kammer 6 ist ein Volumenstrom eines am Einlass eintretenden Stoffs steuerbar. Bei diesem Stoff kann es sich insbesondere um Parawasserstoffgas handeln. Auch kann mittels der Ventile 40 eine Flüssigkeit 12 die erste Kammer 4 zugeleitet werden und eine weitere Flüssigkeit 13 in die zweite Kammer 6.

Ein weiteres Ventil 43 ist zwischen der ersten Kammer 4 und der zweiten Kammer 6 angeordnet. Wird dieses Ventil 43 geschlossen, so kann beispielsweise die erste Kammer 4 als Vorkammer verwendbar sein. Befindet sich beispielsweise in der ersten Kammer 4 Wasser und wird über den Einlass 48 Parawasserstoffgas zugeleitet, so kann in der ersten Kammer 4 eine Wasserstofflösung 14 vorgesättigt werden. Ist die zweite Kammer 6 mit einer weiteren Flüssigkeit 13 befüllt, die einem Katalysator 22 und die Tracer-Moleküle 20 enthält, und ist in der ersten Kammer 4 ein höherer Druck eingestellt als in der zweiten Kammer 6, so fließt bei Öffnen des Ventils 43 die Wasserstofflösung 14 aus der ersten Kammer 4 in die zweite Kammer 6, so dass dort die beiden Flüssigkeiten 12, 13 miteinander vermischt werden und hierdurch das Reaktionsgemisch 10 entsteht. Durch das Vermischen werden die Tracer-Moleküle 20 mit dem Wasserstoff 16 aus der Wasserstofflösung 14 in Kontakt gebracht. Infolge der durch die Vermischung ausgelösten Hydrierungsreaktion werden die Tracer-Moleküle 20 hydriert.

Über die an den Auslässen 28 und 29 angeordneten Ventil 41 kann ein Stoff aus den Kammern 4 oder 6 ausgebracht werden. Zugleich können diese Ventile 41 auch einem Druckausgleich oder einer Druckeinstellung dienen. Nicht näher dargestellt ist, dass auch an der Leitung 36 ein Ventil 45 angeordnet ist. Wird dieses Ventil 45 geöffnet, so können über diese Leitung 36 hydrierte und/oder hyperpolarisierte Tracer-Moleküle 20 einer weiteren Verwendung zugeführt werden.

Fig. 5 zeigt ein alternatives Ausführungsbeispiel einer erfindungsgemäßen Vorrichtung 1 zur Hydrierung von hyperpolarisierbaren Tracer-Molekülen 20, 88 bis 98 für die Magnetresonanzbildgebung. Im Unterschied zu der in Fig. 4 gezeigten Vorrichtung 1 hat der Reaktor 2 in dem alternativen Ausführungsbeispiel lediglich eine einzige erste Kammer 4. Diese Kammer 4 hat eine Zuleitung 26 sowie eine weitere ausgehende Leitung 36. An jedem dieser Anschlüsse ist jeweils ein Ventil 40, 41 angeordnet.

Weiter ist an einer transparenten Wand der Kammer 4 ein Emitter 46 angeordnet, mit dem elektromagnetische Wellen einer bestimmten Wellenlänge wie etwa einer bestimmten Farbe im optischen Bereich oder UV-Licht mit einer Wellenlänge, die beispielsweise einen Wert zwischen von 200nm bis 250nm aufweisen kann, in die Kammer 4 emittierbar sind. Wird nun die Kammer 4 mit allen für die Hydrierungsreaktion erforderlichen Stoffen befüllt, so dass in der Kammer 4 bereits das Reaktionsgemisch 10 vorliegt, und wird ein mit Licht schaltbarer Katalysator 22 verwendet, so kann durch Aktivieren des Emitters 46 der Katalysator 22 geschaltet werden und somit die Hydrierungsreaktion ausgelöst werden. In einem alternativen Ausführungsbeispiel ist die Wand schalldurchlässig ausgebildet und der Emitter 46 emittiert Schallwellen, welche einen hierauf ansprechenden Katalysator 22 aktivieren. In einem weiteren alternativen Ausführungsbeispiel werden anstelle eines durch Bestrahlung aktivierbaren Katalysators 22 die Tracer-Moleküle 88 durch Bestrahlung mit elektromagnetischer Strahlung einer geeigneten Wellenlänge, wie beispielsweise mit UV-Licht, in einen angeregten Elektronenzustand versetzt.

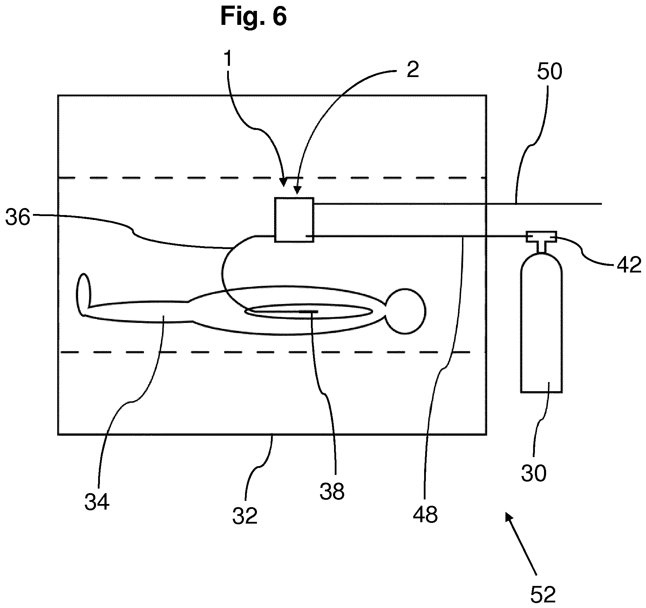

Fig. 6 zeigt eine für eine MR-Messung an einem Patienten 34 vorbereitete Messvorrichtung 52, in der eine Vorrichtung 1 wie sie in Fig. 4 oder Fig. 5 gezeigt ist, angeordnet ist.

Die Messvorrichtung 52 umfasst eine Flasche 30, in der Parawasserstoffgas gelagert ist. Das Parawasserstoffgas kann über das Ventil 42 und den Einlass 48 in den Reaktor 2 gelangen. Weiter sind Mittel ausgebildet, mit denen auch die Tracer-Moleküle 20 und der Katalysator 22 in den Reaktor 2 gelangen können. In dem Reaktor 2 können sodann die Tracer-Moleküle 20 wie zuvor beschrieben hydriert werden. Der Reaktor 2 ist angeordnet in einem Messvolumen eines MR-Tomographen 32. Der MR-Tomograph 32 dient zugleich als Polarisator zur Hyperpolarisierung der hydrierten Tracer-Moleküle 20, was wie bereits zuvor beschrieben erfolgen kann. Der Reaktor 2 ist sodann über die Leitung 36 in einem Injektionskatheter verbunden, an dessen Ende eine an einen Patienten 34 angelegte Kanüle 38 angeordnet ist. Hierdurch kann die Lösung mit hyperpolarisierten Tracer-Molekülen 20 als Kontrastmittel dem Patienten 34 injiziert werden, so dass ein Kontrastmittel zur nachfolgenden MR-Messung zur Verfügung steht.

Zusammenfassend wird bei einem Verfahren 100 bis 192, bei dem hyperpolarisierbare Tracer-Moleküle 20, 88 bis 98 für die Magnetresonanzbildgebung hydriert und gegebenenfalls anschließend auch hyperpolarisiert werden, vorgeschlagen, in einem ersten Verfahrensschritt 104 eine Wasserstofflösung 10, 12, 14 mit einem Sättigungsfaktor von mindestens 50% herzustellen und erst in einem nachfolgenden zweiten Verfahrensschritt 106 die Hydrierungsreaktion 186 bis 190 auszulösen. Die Erfindung umfasst ferner eine Vorrichtung 1, mit der das erfindungsgemäße Verfahren 100 bis 192 ausführbar ist.

### Bezugszeichenliste

- 1: Vorrichtung
- 2: Reaktor
- 4: eine erste Kammer
- 6: eine zweite Kammer
- 10: Reaktionsgemisch
- 12: Flüssigkeit
- 13: weitere Flüssigkeit
- 14: Wasserstofflösung
- 16: Wasserstoff
- 18: Wasserstoffgas
- 20: hyperpolarisierbares Tracer-Molekül für die MR-Bildgebung
- 22: Katalysator
- 24: ein erstes Füllvolumen
- 26: Zuleitung von 4
- 27: Zuleitung von 6
- 28: Auslass von 4
- 29: Auslass von 6
- 30: Flasche
- 32: MR-Tomograph
- 34: Patient
- 36: Leitung
- 37: Verbindungsleitung
- 38: Kanüle
- 40: Ventil
- 41: weiteres Ventil
- 42: Ventil von 30
- 43: Ventil von 37
- 44: Heizelement
- 45: Ventil von 36
- 46: Emitter
- 48: Einlass
- 50: Auslass
- 52: Messvorrichtung
- 80: Komplex
- 82: weiterer Komplex
- 83: Wasserstoffgas
- 84: Norbornadien
- 86: Katalysator
- 88: ungesättigtes Tracer-Molekül
- 90: weiterer Komplex
- 92: weiterer Komplex
- 94: weiterer Komplex
- 96: hydriertes Tracer-Molekül
- 97: angeregtes Tracer-Molekül
- 98: hyperpolarisiertes Tracer-Molekül
- 99: UV-Licht
- 100: Initialisierung
- 102: Bereitstellen von Parawasserstoffgas
- 104: Herstellen von Stoffgemischen
- 106: Vermischen der Stoffgemische
- 108: Anwendung einer SOT-Sequenz
- 110: Verfahrensbeendigung
- 180: Austauschreaktion
- 182: Abspaltungsreaktion
- 184: Additionsreaktion
- 186: Additionsreaktion
- 188: Hydrierungsreaktion
- 190: Abspaltungsreaktion
- 192: Hyperpolarisierung mittels Transfers von Spinordnung
- 202: Herstellung eines Reaktionsgemischs
- 204: Bestrahlung
- 206: Hydrierungsreaktion
- 208: Hyperpolarisierung mittels Transfers von Spinordnung

## Patentansprüche

1. Verfahren (100 bis 206), bei dem hyperpolarisierbare Tracer-Moleküle (20, 88 bis 98) für die Magnetresonanzbildgebung hydriert werden, wobei die Tracer-Moleküle (20, 88 bis 98) mit Wasserstoff (14, 16, 18, 83) in einem Reaktionsgemisch (10, 12, 14) in Kontakt gebracht werden und eine Hydrierungsreaktion in dem Reaktionsgemisch (10, 12, 14) ausgelöst wird, **dadurch gekennzeichnet, dass** in einem ersten Verfahrensschritt (104, 202) der Wasserstoff (14, 16, 18, 83) in einer Flüssigkeit (10, 12, 14) gelöst wird, so dass eine Wasserstofflösung (10, 12, 14) mit einem Sättigungsfaktor von mindestens 50% hergestellt wird, und dass in einem nachfolgenden zweiten Verfahrensschritt (106, 204) die Hydrierungsreaktion (186 bis 190, 206) ausgelöst wird.

2. Verfahren nach dem vorangehenden Anspruch, **dadurch gekennzeichnet, dass** der Sättigungsfaktor mindestens 80%, vorzugsweise mindestens 90% oder 95%, besonders vorzugsweise 100%, beträgt.

3. Verfahren nach einem der vorangehenden Ansprüche, **dadurch gekennzeichnet, dass** das Reaktionsgemisch (10, 12, 14) in dem ersten Verfahrensschritt (104, 202) hergestellt wird oder dass das Reaktionsgemisch (10, 12, 14) in dem zweiten Verfahrensschritt (106, 204) hergestellt wird, insbesondere wobei die Hydrierungsreaktion (186 bis 190, 206) unmittelbar durch die Herstellung des Reaktionsgemischs (10, 12, 14) ausgelöst wird.

4. Verfahren nach einem der vorangehenden Ansprüche, **dadurch gekennzeichnet, dass** die Hydrierungsreaktion (186 bis 190, 206) ausgelöst wird, indem in dem zweiten Verfahrensschritt (106, 204) die Wasserstofflösung (12, 14) mit einem weiteren Reaktionsstoff (20, 22, 86 bis 98) oder Gemisch von Reaktionsstoffen (20, 22, 86 bis 98) vermischt wird, und/oder dass die Hydrierungsreaktion (186 bis 190, 206) ausgelöst wird, indem in dem zweiten Verfahrensschritt (106, 204) ein schaltbarer Katalysator (22, 86) aktiviert wird.

5. Verfahren nach einem der vorangehenden Ansprüche, **dadurch gekennzeichnet, dass** die Hydrierungsreaktion (186 bis 190, 206) ausgelöst wird, indem in dem zweiten Verfahrensschritt (106, 204) das Reaktionsgemisch (10, 12, 14) bestrahlt wird (204), insbesondere wobei durch die Bestrahlung (204) die Tracer-Moleküle (20, 88 bis 98) in einen angeregten Elektronenzustand versetzt werden.

6. Verfahren nach einem der vorangehenden Ansprüche, **dadurch gekennzeichnet, dass** die Flüssigkeit (10, 12, 14) mit einem Stoff angereichert wird, der die Aufnahmefähigkeit der Flüssigkeit (10, 12, 14) für Wasserstoff (14, 16, 18) erhöht, und/oder dass dem Reaktionsgemisch (10, 12, 14) ein H2-Donor zugegeben wird, insbesondere wobei der H2-Donor nach der Hydrierungsreaktion (186 bis 190, 206) wieder entfernt wird.

7. Verfahren nach einem der vorangehenden Ansprüche, **dadurch gekennzeichnet, dass** eine oder die weitere mit einem oder dem Katalysator (22, 86) in Kontakt stehenden Flüssigkeit (13), mit einem Wasserstoffgas (18, 83) vorgesättigt wird, insbesondere wobei das Wasserstoffgas (18, 83) normales Wasserstoffgas (18, 83) ist.

8. Verfahren nach einem der vorangehenden Ansprüche, **dadurch gekennzeichnet, dass** in dem ersten Schritt das Wasserstoffgas (18, 83) mit einem gegenüber der Atmosphäre erhöhten Druck beaufschlagt wird, insbesondere wobei die Hydrierungsreaktion (186 bis 190, 206) unter erhöhtem Druck erfolgt, der vorzugsweise geringer ist als der Druck des Wasserstoffgases (18, 83).

9. Verfahren nach einem der vorangehenden Ansprüche, **dadurch gekennzeichnet, dass** die unter erhöhtem Druck ablaufende Hydrierungsreaktion (186 bis 190, 206) unterhalb einer tatsächlichen Siedetemperatur und oberhalb einer Atmosphärendruck-Siedetemperatur, insbesondere oberhalb von 100°C, erfolgt.

10. Verfahren nach einem der vorangehenden Ansprüche, **dadurch gekennzeichnet, dass** bei oder nach Auslösen der Hydrierungsreaktion (186 bis 190, 206) der Druck in einer Kammer (4, 6) eines Reaktors (2), in der die Hydrierungsreaktion (186 bis 190, 206) stattfindet, verringert wird, sodass Wasserstoff (14, 16, 18, 83) infolge Übersättigung unter Blasenbildung austritt und/oder sodass eine Siedetemperatur eines Stoffs (12, 13, 14), welcher dem Reaktionsgemisch (10, 12, 14) zugegeben ist, und/oder eine Siedetemperatur des Reaktionsgemischs (10, 12, 14) überschritten wird.

11. Verfahren nach einem der vorangehenden Ansprüche, **dadurch gekennzeichnet, dass** nach Auslösen der Hydrierungsreaktion (186 bis 190, 206) das Reaktionsgemisch (10, 12, 14) mit einem Wasserstoffgas (18, 83) in Kontakt gebracht wird.

12. Verfahren (100 bis 208), bei dem Tracer-Moleküle (20, 88 bis 98) für die Magnetresonanzbildgebung hyperpolarisiert werden unter Anwendung eines Verfahrens (100 bis 206) nach einem der vorangehenden Ansprüche.

13. Vorrichtung (1) zur Hydrierung von hyperpolarisierbaren Tracer-Molekülen (20, 88 bis 98) für die Magnetresonanzbildgebung, mit einem Reaktor (2) zur Hydrierung der Tracer-Moleküle (20, 88 bis 98), wobei der Reaktor (2) eine ein erstes Füllvolumen (24) aufweisende erste Kammer (4) umfasst, welche eine Zuleitung (26) für Wasserstoffgas (18, 83) und/oder für eine Flüssigkeit (10, 12, 13, 14) aufweist, **dadurch gekennzeichnet, dass** Mittel (2, 4, 6, 24 bis 30, 36, 40) ausgebildet sind, mit denen das Verfahren (100 bis 206) nach einem der Ansprüche 1 bis 12 ausführbar ist, und/oder dass die Vorrichtung (1) eingerichtet ist, das Verfahren (100 bis 206) nach einem der Ansprüche 1 bis 12 auszuführen.

## Claims

1. Method (100 to 206) in which hyperpolarizable tracer molecules (20, 88 to 98) are hydrogenated for magnetic resonance imaging, wherein the tracer molecules (20, 88 to 98) are brought into contact with hydrogen (14, 16, 18, 83) in a reaction mixture (10, 12, 14) and a hydrogenation reaction is initiated in the reaction mixture (10, 12, 14), **characterized in that** in a first method step (104, 202) the hydrogen (14, 16, 18, 83) is dissolved in a liquid (10, 12, 14), resulting in the preparation of a hydrogen solution (10, 12, 14) having a saturation factor of at least 50%, and that in a subsequent second method step (106, 204) the hydrogenation reaction (186 to 190, 206) is initiated.

2. Method according to the preceding claim, **characterized in that** the saturation factor is at least 80%, preferably at least 90% or 95%, more preferably 100%.

3. Method according to either of the preceding claims, **characterized in that** the reaction mixture (10, 12, 14) is prepared in the first method step (104, 202) or that the reaction mixture (10, 12, 14) is prepared in the second method step (106, 204), in particular wherein the hydrogenation reaction (186 to 190, 206) is initiated directly by the preparation of the reaction mixture (10, 12, 14).

4. Method according to any of the preceding claims, **characterized in that** the hydrogenation reaction (186 to 190, 206) is initiated by the hydrogen solution (12, 14) being mixed with a further reactant (20, 22, 86 to 98) or mixture of reactants (20, 22, 86 to 98) in the second method step (106, 204), and/or that the hydrogenation reaction (186 to 190, 206) is initiated by a switchable catalyst (22, 86) being activated in the second method step (106, 204).

5. Method according to any of the preceding claims, **characterized in that** the hydrogenation reaction (186 to 190, 206) is initiated by the reaction mixture (10, 12, 14) being irradiated (204) in the second method step (106, 204), in particular wherein the tracer molecules (20, 88 to 98) are brought to an excited electronic state by the irradiation (204).

6. Method according to any of the preceding claims, **characterized in that** the liquid (10, 12, 14) is enriched with a substance that increases the uptake capacity of the liquid (10, 12, 14) for hydrogen (14, 16, 18), and/or that an H2 donor is added to the reaction mixture (10, 12, 14), in particular wherein the H2 donor is removed again after the hydrogenation reaction (186 to 190, 206).

7. Method according to any of the preceding claims, **characterized in that** a liquid or the further liquid (13) in contact with a catalyst or the catalyst (22, 86) is presaturated with a hydrogen gas (18, 83), in particular wherein the hydrogen gas (18, 83) is normal hydrogen gas (18, 83).

8. Method according to any of the preceding claims, **characterized in that** in the first step the hydrogen gas (18, 83) is pressurized to above atmospheric pressure, in particular wherein the hydrogenation reaction (186 to 190, 206) is carried out under elevated pressure that is preferably lower than the pressure of the hydrogen gas (18, 83).

9. Method according to any of the preceding claims, **characterized in that** the hydrogenation reaction (186 to 190, 206) taking place under elevated pressure is carried out below an actual boiling temperature and above a boiling temperature at atmospheric pressure, in particular above 100°C.

10. Method according to any of the preceding claims, **characterized in that**, during or after the initiation of the hydrogenation reaction (186 to 190, 206), the pressure in a chamber (4, 6) of a reactor (2) in which the hydrogenation reaction (186 to 190, 206) takes place is reduced, resulting in the escape of hydrogen (14, 16, 18, 83) as a consequence of supersaturation, with attendant bubble formation, and/or resulting in a boiling temperature of a substance (12, 13, 14) that is added to the reaction mixture (10, 12, 14) and/or a boiling temperature of the reaction mixture (10, 12, 14) being exceeded.

11. Method according to any of the preceding claims, **characterized in that**, after initiation of the hydrogenation reaction (186 to 190, 206), the reaction mixture (10, 12, 14) is brought into contact with a hydrogen gas (18, 83).

12. Method (100 to 208) in which tracer molecules (20, 88 to 98) for magnetic resonance imaging are hyperpolarized using a method (100 to 206) according to any of the preceding claims.

13. Device (1) for the hydrogenation of hyperpolarizable tracer molecules (20, 88 to 98) for magnetic resonance imaging, having a reactor (2) for hydrogenating the tracer molecules (20, 88 to 98), wherein the reactor (2) includes a first chamber (4) having a first fill-volume (24), which has a feed line (26) for hydrogen gas (18, 83) and/or for a liquid (10, 12, 13, 14), **characterized in that** means (2, 4, 6, 24 to 30, 36, 40) are provided with which the method (100 to 206) according to any of Claims 1 to 12 can be executed, and/or that the device (1) is configured to execute the method (100 to 206) according to any of Claims 1 to 12.

## Revendications

1. Procédé (100 à 206) par lequel des molécules de traçage hyperpolarisables (20, 88 à 98) pour l'imagerie par résonance magnétique sont hydrogénées, les molécules de traçage (20, 88 à 98) étant mises en contact avec de l'hydrogène (14, 16, 18, 83) dans un mélange réactionnel (10, 12, 14) et une réaction d'hydrogénation étant déclenchée dans le mélange réactionnel (10, 12, 14), **caractérisé en ce que,** dans une première étape de procédé (104, 202), l'hydrogène (14, 16, 18, 83) est dissous dans un liquide (10, 12, 14), de sorte qu'une solution hydrogénée (10, 12, 14) dotée d'un facteur de saturation d'au moins 50% est fabriquée, et que, dans une deuxième étape de procédé (106, 204), la réaction d'hydrogénation (186 à 190, 206) est déclenchée.

2. Procédé selon la revendication précédente, **caractérisé en ce que** le facteur de saturation est d'au moins 80%, de préférence d'au moins 90% ou 95%, de manière particulièrement préférée de 100%.

3. Procédé selon une des revendications précédentes, **caractérisé en ce que** le mélange réactionnel (10, 12, 14) est fabriqué dans la première étape de procédé (104, 202) ou que le mélange réactionnel (10, 12, 14) est fabriqué dans la deuxième étape de procédé (106, 204), la réaction d'hydrogénation (186 à 190, 206) étant en particulier déclenchée directement par la fabrication du mélange réactionnel (10, 12, 14).

4. Procédé selon une des revendications précédentes, **caractérisé en ce que** la réaction d'hydrogénation (186 à 190, 206) est déclenchée du fait que, dans la deuxième étape de procédé (106, 204), la solution hydrogénée (12, 14) est mélangée avec un autre réactif (20, 22, 86 ou 98) ou mélange de réactifs (20, 22, 86 ou 98) et/ou que la réaction d'hydrogénation (186 à 190, 206) est déclenchée du fait que, dans la deuxième étape de procédé (106, 204), un catalyseur commutable (22, 86) est activé.

5. Procédé selon une des revendications précédentes, **caractérisé en ce que** la réaction d'hydrogénation (186 à 190, 206) est déclenchée du fait que, dans la deuxième étape de procédé (106, 204), le mélange réactionnel (10, 12, 14) est irradié (204), la molécule de traçage (20, 88 à 98) étant en particulier mise par l'irradiation (204) dans un état électronique excité.

6. Procédé selon une des revendications précédentes, **caractérisé en ce que** le liquide (10, 12, 14) est enrichi avec une substance qui augmente la capacité d'absorption d'hydrogène (14, 16, 18) du liquide (10, 12, 14) et/ou qu'au mélange réactionnel (10, 12, 14) est ajouté un donneur de H2, le donneur de H2 étant en particulier retiré après la réaction d'hydrogénation (186 à 190, 206).

7. Procédé selon une des revendications précédentes, **caractérisé en ce qu'**un ou l'autre liquide (13) en contact avec un ou le catalyseur (22, 86) est présaturé avec un hydrogène gazeux (18, 83), l'hydrogène gazeux (18, 83) étant en particulier de l'hydrogène gazeux (18, 83) normal.

8. Procédé selon une des revendications précédentes, **caractérisé en ce que**, dans la première étape, l'hydrogène gazeux (18, 83) est soumis à une pression élevée par rapport à l'atmosphère, la réaction d'hydrogénation (186 à 190, 206) se déroulant en particulier sous une pression élevée qui est de préférence inférieure à la pression de l'hydrogène gazeux (18, 83).

9. Procédé selon une des revendications précédentes, **caractérisé en ce que** la réaction d'hydrogénation (186 à 190, 206) se déroulant sous une pression élevée a lieu au-dessous d'une température d'ébullition effective et au-dessus d'une température d'ébullition à la pression atmosphérique, en particulier au-dessus de 100 °C.

10. Procédé selon une des revendications précédentes, **caractérisé en ce que**, pendant ou après le déroulement de la réaction d'hydrogénation (186 à 190, 206), la pression dans une chambre (4, 6) d'un réacteur (2) dans laquelle la réaction d'hydrogénation (186 à 190, 206) a lieu est réduite, de sorte que de l'hydrogène (14, 16, 18, 83) s'échappe en formant des bulles suite à la sursaturation et/ou de sorte qu'une température d'ébullition d'une substance (10, 12, 14) qui est ajoutée et/ou une température d'ébullition du mélange réactionnel (10, 12, 14) sont excédées.

11. Procédé selon une des revendications précédentes, **caractérisé en ce qu'**après le déroulement de la réaction d'hydrogénation (186 à 190, 206), le mélange réactionnel (10, 12, 14) est mis en contact avec un hydrogène gazeux (18, 83).

12. Procédé (100 à 208) par lequel des molécules de traçage (20, 88 à 98) pour l'imagerie par résonance magnétique sont hyperpolarisées en appliquant un procédé (100 à 206) selon une des revendications précédentes.

13. Dispositif (1) d'hydrogénation de molécules de traçage hyperpolarisables (20, 88 à 98) pour l'imagerie par résonance magnétique, avec un réacteur (2) pour l'hydrogénation des molécules de traçage (20, 88 à 98), le réacteur (2) comprenant une première chambre (4) présentant un premier volume de remplissage (24), laquelle comporte une conduite d'amenée (26) pour de l'hydrogène gazeux (18, 83) et/ou pour un liquide (10, 12, 13, 14), **caractérisé en ce que** sont prévus des moyens (2, 4, 6, 24 à 30, 36, 40) avec lesquels le procédé (100 à 206) selon une des revendications 1 à 12 peut être mis en œuvre et/ou que le dispositif (1) est équipé pour mettre en œuvre le procédé (100 à 206) selon les revendications 1 à 12.
